(19)

**Europäisches Patentamt**
**European Patent Office**
**Office européen des brevets**

(11) **EP 1 999 469 B1**

(12) **EUROPEAN PATENT SPECIFICATION**

(45) Date of publication and mention
of the grant of the patent:
**22.07.2009 Bulletin 2009/30**

(51) Int Cl.:
*G01N 33/50* *(2006.01)*     *G01N 33/68* *(2006.01)*

(21) Application number: **07727512.1**

(22) Date of filing: **29.03.2007**

(86) International application number:
**PCT/EP2007/053040**

(87) International publication number:
**WO 2007/113211 (11.10.2007 Gazette 2007/41)**

(54) **IG-ASSAY**

IG-ASSAY

ANALYSE D'IMMUNOGLOBULINE

(84) Designated Contracting States:
**AT BE BG CH CY CZ DE DK EE ES FI FR GB GR
HU IE IS IT LI LT LU LV MC MT NL PL PT RO SE
SI SK TR**

(30) Priority: **30.03.2006 US 787468 P
30.03.2006 SE 0600729**

(43) Date of publication of application:
**10.12.2008 Bulletin 2008/50**

(73) Proprietor: **GYROS PATENT AB
751 83 Uppsala (SE)**

(72) Inventors:
• **INGANÄS, Mats
S-757 55 Uppsala (SE)**
• **ECKERSTEN, Ann
SE - 756 53 Uppsala (SE)**

(74) Representative: **Franks, Barry Gerard
BRANN AB
Uppsala Office
Box 171 92
104 62 Stockholm (SE)**

(56) References cited:
• **JANSSON B ET AL: "All individual domains of
staphylococcal protein A show Fab binding"
FEMS IMMUNOLOGY AND MEDICAL
MICROBIOLOGY, ELSEVIER SCIENCE B.V.,
AMSTERDAM, NL, vol. 20, no. 1, January 1998
(1998-01), pages 69-78, XP002284946 ISSN:
0928-8244 cited in the application**
• **BRANDES W ET AL: "SPECIFIC FLOW
INJECTION SANDWICH BINDING ASSAY FOR
IGG USING PROTEIN A AND A FUSION PROTEIN"
ANALYTICAL CHEMISTRY, AMERICAN
CHEMICAL SOCIETY. COLUMBUS, US, vol. 65,
no. 23, 1 December 1993 (1993-12-01), pages
3368-3371, XP000416621 ISSN: 0003-2700**
• **STRANDBERG L ET AL: "EXPRESSION AND
CHARACTERIZATION OF A TRIPARTITE FUSION
PROTEIN CONSISTING OF CHIMERIC IGG-
BINDING RECEPTORS AND BETA
GALACTOSIDASE" JOURNAL OF
BIOTECHNOLOGY, ELSEVIER SCIENCE
PUBLISHERS, AMSTERDAM, NL, vol. 13, no. 1,
January 1990 (1990-01), pages 83-96,
XP009077811 ISSN: 0168-1656**

**EP 1 999 469 B1**

**Description**

**TECHNICAL FIELD**

The present invention relates to a method for the quantitative determination of an immunoglobulin (Ig) of a certain class (IgX), such as IgG, in a liquid sample. The method is a sandwich assay and thus comprises the formation of an at least ternary affinity complex that comprises IgX sandwiched between two affinity reactants 1 and 2 (R1 and R2).

**BACKGROUND TECHNOLOGY**

One of the more promising new therapeutic principles for treatment of e.g. cancer or inflammatory diseases is the use of recombinant monoclonal antibodies, typically of the IgG class. Although a number of alternative molecular configurations may be used the most common type of therapeutic agent is still IgG, typically human, produced by recombinant techniques mostly containing at least a part of the Ig constant domain. The number of monoclonal products approved for treatment of humans is still rather limited but the use is increasing due to sometimes spectacular clinical results. The number of product candidates in various stage of clinical development is probably exceeding 500.
The bioprocess utilized for production of therapeutic monoclonal antibody products has to be optimized both for quality and productivity. Selection of clones with the highest Ig producing capacity becomes an essential part of product development. Hence, access to convenient, robust and generic analytical methods for accurate quantification and characterization of this kind of Ig-products will become indispensable tools that can be used in product development, not only for single Ig candidates but generally applicable for quantification of a wide range of Ig products.
In cell supernatants during the early phase of development the concentration of the desired Ig product may vary from 20-2000 mg/L. Later, during scale up and after purification the concentration of the Ig product may be in the interval of 1-100 g/L. Thus sample dilutions will be required to cover this wide range, However, there is a strong desire to keep dilution factors to a minimum, both for convenience but also to avoid introduction of errors due to serial dilutions. Thus it would be desirable to assay non-purified samples and other samples in essentially un-diluted form, i.e. samples diluted less than 1:10, such as less than 1:5 or less than 1:2. An assay that covers a concentration range of at least one, such as at least two, three or more orders of magnitude, for instance starting between 1 mg/L and 10 mg/L or between 10 mg/L and 100 mg/L or between 100 mg/L and 1000 mg/L would be very attractive for this purpose.
There are at least three factors that will affect the assay range a) affinity and selectivity of R1, for instance as an immobilized capture reagent, and possible also the the affinity and selectivity of R2, for instance as a detectable reactant, b) the volume of the sample containing the Ig analyte, and c) capacity of the solid phase for capturing the Ig analyte (if a solid phase is used for immobilizing R1).
The inventor has experience from assaying recombinant IgG products using monoclonal and polyclonal anti-IgGs and their antigen binding fragments as one or both of R1 and R2 including that the remaining one has been an IgG-binding recombinant construct of an IgG-binding polypeptide of microbial origin (protein G and protein A). See for instance (Z-fragment of protein A as R1 in combination with a labeled Fab2 fragment as R2). For variants in which both R1 and R2 were monoclonal anti-IgG, the working range was 1 µg/L-10mg/L of analyte. Unfortunately these assays and particularly the antibody seem to react with different recombinant IgG molecules (analytes) in a variable way. The implication is that each analyte will require its own reference in order to accurately quantify different recombinant IgG analytes. The mechanism behind this problem is unclear at the moment but variation in the expression of relevant epitopes on the Ig-analytes might be one explanation. It would be more attractive to only use reagents that react with the analytes in a more generic fashion, for instance using bacterially originating Ig-binding molecules like protein A or protein G including their recombinant derivatives including fragments, mutants and conjugates.
The use of IgG-binding molecules derived from microorganisms as both R1 and R2 would require that the binding sites utilized on the IgG analyte should be more or less repetitive at least if the actually used R1 and R2 have the same binding specificity for the analyte. The question about a) the number of binding sites on IgG for a bacterially derived IgG-binding molecule, and b) the ability of two such sites to simultaneously bind two bacterially derived IgG-binding molecules has been open for at least two decades. Most likely this has been the reason for the fact that the development of IgG assays based on R1 and R2 of the same binding specificity has been hampered and never seems to have been reduced to practice.
The goal of the invention is to provide generic Ig assays that are specific to a certain Ig class, such as IgA, IgD, IgE, IgM etc, and easily can be adapted to the concentration ranges that are within the ranges discussed above without significant dilution of original samples. Other goals are to provide rapid and robust Ig assays that are easy to perform and automate with a high productivity with respect to the number of assays performed per time unit. A very important goal is to create a generic assay for the quantification of recombinant antigen specific IgG antibody products that are monoclonal and are present in various kinds of cell culture media or work up liquid preparations from cell cultures.

## FIGURES

**Figure 1** gives a set of microchanel structures of a device used in the experimental part.
**Figures 2-6** present the results of the experimental part. See further the experimental part.
**Figures 7-10** depicts the results of measuring monomeric vs. polymeric IgG.

## THE INVENTION

The present inventor has realized that sandwich immunoglobulin assays in which both of the reactants R1 and R2 are based on microbial Ig-binding peptide sequences are highly feasible and advantageous with respect to at least recombinantly produced Ig products.

The invention is a method for the quantitative determination of an immunoglobulin (Ig) of a certain class (IgX) in a liquid sample. He method comprises one, two or more steps for forming an at least ternary affinity complex in which IgX is sandwiched between the two affinity reactants 1 and 2 (R1 and R2, respectively). The characterizing feature is that each of R1 and R2 comprises a peptide sequence (seq1 and seq2, respectively) that is derived from a microbial polypeptide (P1 and P2, respectively) that is capable of affinity binding to a binding site that is located within a constant part of said IgX. The two peptide sequences are responsible for the simultaneous binding of R1 and R2 to the Ig-analyte in the ternary sandwich complex, which means that the binding site for R1 is separated (roomly spaced) from the binding site for R2.

The Ig-binding sequence in R1 and R2, respectively, is the same as in the native microbial polypeptide or a derivative thereof that retains at least some of the native Ig-binding ability of the microbial polypeptide (P1 and P2, respectively). The term derivative includes mutated forms and fragments that retain IgG binding. P1 may be equal to P2, i.e. the Ig-binding sequences in R1 and R2 derives from the same microbial polypeptide.

In preferred embodiments R1 is immobilized or immobilizable to a solid phase and R2 is analytically detectable/ measurable.

Microbial polypeptides that are capable of binding to constant parts of immunoglobulin, for instance of one or more classes or subclasses, are well known in the field.

The most studied polypeptides of this kind are protein A from *Staphylococcus aureus,* protein G from *Streptococcus* Group C and G Fcg, protein H from *streptococcus pyogenes* Fcg, and protein L from *Peptostreptococcus magnus.* Other similar proteins are protein MAG and ZAG from other streptococci.

Each of R1 and R2 derives its Ig-binding activity from a native Ig-binding microbial polypeptide including the polypeptide as such or derivatives thereof with due care taken for the affinity and specificity in binding required for the particular Ig analyte to be determined, including such things as the concentration range at issue..

An immunoglobulin molecule contains two heavy (H) chains and two light (L) chains. Each chain contains a variable (V) domain that is responsible for antigen-binding and a constant (C) domain that is responsible for various effector mechanisms that an immunoglobulin can participate in. Within the variable domain of each chain there are hyper variable regions such as CDR1-3 and regions that are more or less conserved, so called framework structures, such as FR1-3. The constant domain comprises regions that in the heavy chain of IgG, IgA, IgM and IgE are called: $C_H1$, $C_H2$ and $C_H3$.

In the context of the invention a constant part of an immunoglobulin comprises the constant domain as well as conserved parts of the variable domain. The term immunoglobulin also includes corresponding protein in animals other than mammals, e.g. avians, amphibians etc.

Protein A primarily reacts with IgG via the classical mechanism through the interface between $C_H2$-$C_H3$ and via the variable domain belonging to the $V_H$III class. It is well established that protein A interacts with three of the four subclasses of human IgG excluding IgG3. Thirty to fifty percent of human polyclonal immunoglobulins carry the $V_H$III class and is not generically expressed on all IgG molecules. The two types of reactivity are expressed on all of the five immunoglobulin-binding regions of protein A (regions C, B, A, D and E). A recombinant fragment from one of the regions (region B) has been mutated to loose the $V_H$III binding activity but retained the $C_H2$-$C_H3$ binding activity. This fragment is called fragment Z and is the archetype molecule for an affibody (Nilsson B et al., Protein Eng. 1 (1987) 107-113).

The interaction between the monovalent fragment Z and IgG is in the order of $2x10^7$ $M^{-1}$ (Nilsson J et al., Eur J Biochem 224 (1994) 103-108). The corresponding interaction with $V_H$III is $\approx$ $9x10^6$ $M^{-1}$ (Jansson B et al., FEMS Immunology and Medical Microbiology 20 (1998) 69-78.

Recombinant protein G (devoid of the albumin binding sites in native protein G) carries 3 binding regions for IgG (C1, C2, C3). Each of the regions seems to harbour two different reaction mechanisms for immunoglobulins. One of them interacts with the interface between $C_H2$-$C_H3$ of immunoglobulins. The mechanism of interaction is different from protein A but the interaction of each of them affects the interaction of the other molecule suggesting their sites of interaction on IgG are in close proximity. The other mechanism involves the $C_H1$ domain of IgG. Available literature suggest that the structure represented by the $C_H1$ interaction of the immunoglobulin is the most conserved in the entire Fab fragment (Derrick JP et al., J Mol Biol 243 (1994) 906-918) of not only human IgG but also a number of other species.

The affinity of the interaction between IgG and the C2 fragment is in the order of $1\times10^7$ M$^{-1}$ (Gülich S et al., Protein Eng 15 (2002) 835-842). As of yet the inventor has found no affinity data on the $C_H1$ interaction. However, there are data showing that mouse Fab fragments can be purified on protein G preparatively, and the interaction is strong enough to create crystals for crystallographic analysis of the interaction between the C3 fragment of protein G and $C_H1$ of human IgG (Kelley RF et al., Biochemistry 31 (1992) 5434-5441). In all likelihood the affinity is in the same order of magnitude as for the mechanisms of interaction described here.

There are some studies involving mutations of IgG binding fragments of protein G. In one of them the $C_H2$-$C_H3$ reactivity of the mutant could be completely abolished (Sloan DJ et al., Protein Sci 8 (1999) 1643-1648). Whether the mutant still expresses $C_H1$ reactivity was not tested.

Protein H primarily reacts with $C_H2$-$C_H3$.

Protein L reacts with a framework structure in the variable domain of the Kappa light chain.

Thus, one of R1 and R2 may be capable of affinity binding to a binding site in IgX that is structurally different from the IgX binding site utilized by the other, e.g. R1 and R2 have different binding specificities for IgX. Alternatively, R1 and R2 may be capable of affinity binding to a binding site that is occurring twice or more times in IgX (repetitive binding site), i.e. R1 and R2 have the same binding specificity for IgX. One, two or more of the constant regions in one or more of the Ig chains may define the binding site in IgX, typically one chain, such as a heavy chain. Thus for the measurement of IgA, IgE, IgG and IgM, one or more of the C1, C2 and C3 regions may define the binding sites used by R1 and R2. For the measurement of IgG, the binding sites utilized by R1 or R2 may be defined by $C_H1$ or $C_H2$-$C_H3$, for instance with one of the reactants utilizing a peptide sequence derived from protein G with specificity for $C_H1$ or from protein A with specificity for $C_H2$-$C_H3$, and the other reactant utilizing a peptide sequence derived from protein A, protein G or protein H and with a specificity for $C_H2$-$C_H3$.

A native microbial Ig-binding peptide sequence typically has a dual specificity as discussed above. For R1 and R2 it may be preferred to incorporate forms of the native sequence in which one of the specificities has been abolished, for instance the $C_H1$ or $C_H2$-$C_H3$ specificity if the sequence of a reactant derives from protein G (giving reactant specificity for only $C_H2$-$C_H3$ or $C_H1$, respectively). For reactants R1 and R2 that incorporate a peptide sequence that derive from protein A, the specificity for $V_H$III preferably has been abolished, for instance as in the above-mentioned fragment Z that thus has specificity only for a binding site defined by $C_H2$-$C_H3$. Abolishment of one of the binding specificities is typically accomplished by the proper mutation of the native sequence.

The Ig-binding ability in reactants R1 and R2 may be repetitive, i.e. each of R1 and R2 may comprise one, two, three, four, five or more peptide sequences/fragments that have the same binding specificity for Ig. In addition R1 and R2 typically lack the additional binding specificities that may be present in native forms of the microbial polypeptides from which the Ig-binding sequence(s) of R1 and R2 derive. A reactant comprising a sequence that is derived protein G is thus typically devoid of the albumin-binding ability of native protein G.

## SANDWICH FORMATS

These formats typically utilize non-limiting amounts of R1 and/or R2. For certain variants R1 and/or R2 may be used in limiting amounts. Amounts in this context include concentrations.

As indicated above the binding sites for R1 and R2 on Ig must be sufficiently spaced from each other for simultaneous binding of the two reactants to the Ig-analyte. In preferred variants R1 is immobilized or immobilizable to a solid phase and R2 is analytically detectable/measurable such that it can be discriminated from other reactants that are incorporated into the sandwich complex and/or other constituents that may be present together with this complex during measurement.

Sandwich formats may be divided into two-step formats (sequential formats) and one-step formats (simultaneous formats). For variants utilizing immobilized R1 there are two main two-step variants: a) the forward format in which the first step comprises incubation of an immobilized or immobilizable form of R1 with the Ig-analyte followed by a second step that comprises incubation of the complex formed in the first step with an analytically detectable form of R2, and b) the reverse format in which the first step comprises incubation of an analytically detectable form of R2 with the analyte followed by a second step that comprises incubation of the complex formed in the first step with an immobilized or immobilizable form of R1. A preferred one-step sandwich format comprises incubating an immobilized or immobilizable form of R1, an analytically detectable form of R2, and the Ig-analyte for the formation of the above-mentioned ternary complex without separate preformation of a binary complex that comprises only two reactants (the Ig-analyte plus one of R1 and R2).

The forward format is preferred, in particular the variant utilizing an immobilized form of R1.

For variants involving immobilizable forms of R1, there is typically formed a soluble immobilizable complex that comprises the immobilizable reactant (R1) and the analyte. This complex is typically immobilized via an immobilizing tag on R1 to a solid phase that exhibits an immobilizing group in a separate immobilization step. The immobilizing tag and group are selected according to the same principles as outlined for the immobilization of R1. The immobilization step typically is performed after an immobilizable complex comprising the Ig-analyte and R1 has been formed, which for the forward two-step format means between the first and second step or after the second step, and in the reversed format after the second step. In the simultaneous format the immobilization step is after the single step in which R1, R2 and the Ig-analyte has been incubated with each other.

There are also variants of the above-mentioned basic variants. Such variants typically utilize additional reactants and additional steps, e.g. for measuring a detectable group in R2 (see below), or for immobilization of an immobilizable reactant R1.

In a sandwich format the amount of analyte is preferably determined from the amount of ternary sandwich complex formed, for instance on a solid phase preferably by measuring R2 that has become bound to the solid phase via the ternary complex. In principle measurement of R2 on the solid phase indirectly by measuring R2 remaining in the liquid after formation of the ternary complex may also be feasible.

Known principles are applied in order to find the relation between a measured value for R2 in the sandwich complex and the amount of the Ig-analyte in the sample, typically by comparing a measured value with corresponding values that have been obtained for one or more standard samples.

In advantageous variants of the invention at least the assay reactions that comprise reaction between a soluble immoblizable reactant or complex and a solid phase is performed under flow conditions in the appropriate flow path. In preferred variants, all reactions involving R1, Ig-analyte and R2 are performed within such a flow path. The preferred flow path is microfluidic. Assay reactions that are not performed under flow conditions may take place under static conditions within a flow path used for outside the flow path in the appropriate vessel.

Alternatively all of the assay reactions including the immobilization of R1 or of an immobilizable complex comprising R1 and the Ig-analyte may be performed under static conditions, possibly under turbulent conditions, such as stirring, shaking etc, in the appropriate assay vessel such as a microscale vessel like a microtittre well of a microtitre plate.

**Solid phases and reactant R1 (capturer)**

If used a solid phase is typically in the form of A) a porous bed, for instance a packed bed of particles or a porous monolith, or B) the inner wall of the vessel used for incubation with the Ig-analyte, or C) suspended particles that are capable of sedimenting to a porous bed.

Suitable particles for solid phases are preferably spherical or spheroidal (beads), or non-spherical. Appropriate mean diameters for particles are typically found in the interval of 1-100 $\mu$m with preference for mean diameters that are $\geq$ 5 $\mu$m, such as $\geq$ 10 $\mu$m or $\geq$ 15 $\mu$m and/or $\leq$ 50 $\mu$m. Also smaller particles can be used, for instance with mean diameters down to 0.1 $\mu$m. Diameters refer to the "hydrodynamic" diameters. Particles to be used may be monodisperse (monosized) or polydisperse (polysized) in the same meaning as in WO 02075312 (Gyros AB).

The base material of a solid phase may be made of inorganic and/or organic material. Typical inorganic materials comprise glass. Typical organic materials comprise organic polymers. Polymeric materials comprise inorganic polymers, such as glass and silicone rubber, and organic polymers of synthetic or biological origin (biopolymers). The term "biopolymer" includes semi-synthetic polymers in which there is a polymer backbone derived from a native biopolymer. Appropriate synthetic organic polymers are typically cross-linked and are often obtained by the polymerisation of monomers comprising polymerisable carbon-carbon double bonds. Examples of suitable monomers are hydroxy alkyl acrylates, for instance 2-hydroxyalkyl acrylates such as 2-hydroxyethyl acrylates, and corresponding methacrylates, acryl amides and methacrylamides, vinyl and styryl ethers, alkene substituted polyhydroxy polymers, styrene, etc. Typical biopolymers in most cases exhibit carbohydrate structure, e.g. agarose, dextran, starch etc.

The particles of solid phases may be manufactured from non-magnetic material, e.g. polymeric, into which minor particles of magnetic material, such as ferrite, have been incorporated, or the particles may be based on magnetic particulate material, such as ferrite, that may have been appropriately surface modified.

The solid phases used in the invention are preferably hydrophilic. For porous beds this means that surfaces of the pores of a bed in many cases should have a sufficient wettability for water to be spread by capillarity into the bed (in many cases all throughout the bed) when the bed is in contact with water (absorption). Surfaces of solid phases that are to be in contact with aqueous liquids typically expose a plurality of polar functional groups which each has a heteroatom selected amongst oxygen and nitrogen, for instance. Appropriate functional groups can be selected amongst hydroxy groups, eythylene oxide groups ($-X-[CH_2CH_2O-]_n$ where n is an integer > 1 and X is nitrogen or oxygen), amino groups, amide groups, ester groups, carboxy groups, sulphone groups etc, with preference for those groups that are essentially uncharged independent of pH, for instance within the interval of 2-12.

If the base material of a solid phase material is hydrophobic or not sufficiently hydrophilic, e.g. is based on a styrene (co)polymer, the surfaces that are to be in contact with an aqueous liquid may be hydrophilized. Typical protocols comprise coating with a compound or mixture of compounds exhibiting polar functional groups of the same type as discussed above, treatment by an oxygen plasma etc.

The technique for introducing an immobilised form of reactant R1 on a solid phase typically comprises:

a) firmly attaching a soluble form of R1 to the solid phase, or
b) building an immobilized form of R1 stepwise on the solid phase (solid phase synthesis). Both routes are commonly known in the field. The linkage to the solid phase material may be via covalent bonds, affinity bonds (for instance biospecific affinity bonds), physical adsorption, electrostatic bonds etc.

Alternative a) typically makes use of an immobilizing group on the solid phase and an immobilizing tag on R1 which are mutually reactive with each other to the formation of a bond that resists undesired cleavage under the conditions provided when carrying out the inventive method. The immobilizing group is introduced on the solid phase material before reaction with the immobilizing tag. The immobilizing group and the immobilizing tag define an immobilizing pair.

Covalent immobilization for variant (a) means that the cleavage-resistant bond is covalent. The immobilizing group and the immobilizing tag are typically selected amongst mutually reactive electrophilic and nucleophilic groups, respectively. Examples of groups are for instance given in WO 2004083109, PCT/SE06/000071, and PCT/SE06/000072 (all Gyros AB/Gyros Patent AB).

Immobilization via affinity bonds may utilize an immobilizing affinity pair in which one of the members (immobilized ligand L = immobilizing group) is firmly attached to the solid phase material while the other member (immobilizing binder, B) is part of a conjugate (immobilizing conjugate) that contains a first moiety that comprises binder B (= immobilizing tag) and a second moiety that comprises an Ig-binding peptide sequence. The immobilizing binding pair shall not negatively interfere with the desired binding activity of the reactants used and is in this sense generic (both binder B and the affinity ligand L is generic). Typically preferred immobilizing affinity pairs are biotin-binding compounds such as streptavidin, avidin, neutravidin, anti-biotin antibodies etc and biotin, b) anti-hapten antibodies and the corresponding haptens or antigens, and c) class/subclass-specific antibodies and Igs from the corresponding class.

The term "conjugate" above and in other contexts of the invention refers to covalent conjugates, such as chemical conjugates and recombinantly produced conjugates. A conjugate comprises at least two moieties bound together, typically covalently, via a linker. In the invention one of the moieties may be a polypeptide exhibiting at least one Ig-binding sequence while another one of the moieties may be a generic binder B or an analytically detectable group (see below). If applicable the term also includes so-called native conjugates, i.e. affinity reactants which each exhibits two binding sites that are spaced apart from each other and with affinity directed towards two different molecular entities.

Preferred immobilizing affinity pairs (L and B) typically have affinity constants ($K_{L-B}$ = [L][B]/[L--B]) that are at most equal to the corresponding affinity constant for streptavidin and biotin, or $\leq 10^1$ times or $\leq 10^2$ times or $\leq 10^3$ times larger than this latter affinity constant. This typically will mean affinity constants that roughly are $\leq 10^{-13}$ mole/l, $\leq 10^{-12}$ mole/l, $\leq 10^{-11}$ mole/l and $\leq 10^{-10}$ mole/l, respectively. These affinity constant ranges refer to values obtained by a biosensor (surface plasmon resonance) from Biacore (Uppsala, Sweden), i.e. with the ligand L immobilized to a dextran-coated gold surface.

Ranges for suitable binding capacities for a binder B and measurement of such binding capacities have been given in WO 2004083109, PCT/SE06/000071, and PCT/SE06/000072 (all Gyros AB/Gyros Patent AB).

Immobilizing groups and immobilized capture reactants may be introduced on a solid phase as described in WO 2004083109, PCT/SE06/000071, and PCT/SE06/000072 (all Gyros AB/Gyros Patent AB).

Immobilizing affinity pairs are preferred for immobilization of R1.

**Detectable reactant R2**

An analytically detectable form of R2 typically comprises a moiety 1 in which there is a detectable group and a moiety 2, which comprises one or more Ig-binding peptide sequences. The detectable reactant R2 may thus be a conjugate in which moiety 1 and moiety 2 are firmly attached to each other, preferably by covalent bonds. The detectable group will also be called label.

There are two main types of detectable groups: a) signal-generating groups and b) affinity groups. A signal-generating group may be selected amongst radiation emitting or radiation absorbing groups and groups that in other ways interfere with a given radiation. Particular signal-generating groups are enzymatically active groups such as enzymes, cofactors, substrates, coenzymes etc; groups containing particular isotopes such as radioactive or nonradioactive isotopes; fluorescent and fluorogenic groups; luminescent and luminogenic groups including chemiluminescent and chemiluminogenic groups, bioluminescent and bioluminogenic groups etc; metal-containing groups including groups in which the metal is in ionic form etc. Affinity groups in this context are typically detected by the use of a secondary detectable reactant that is a conjugate between an affinity counterpart to the detectable affinity group and a second detectable group that is different from the detectable group in the detectable reactant, and preferably is a signal-generating group typically in the form of a label. Typical affinity based detectable groups may be selected amongst the individual members of the immobilizing affinity pairs discussed elsewhere in this specification, with the proviso that an affinity based detectable group should not be capable of affinity binding during the method to a member of an immobilizing binding pair if such a pair has been used for the immobilization of R1 (i.e. the capturer) to the solid phase.

**Flow path and flow conditions**

A flow path is preferably of the kind that is present in microfluidic devices, i.e. a microchannel structure fabricated in a substrate and defined by a system of microconduits comprising functional units that enables all of the steps of an assay protocol that are to be carried out in the structure including also transport microconduits between the units. Typical microfluidic devices have for instance been described by Gyros AB/Amersham Biosciences (WO 99055827, WO 99058245, WO 02074438, WO 02075312, WO 03018198 (US 20030044322) etc); Tecan/Gamera Bioscience (WO 01087487, WO 01087486, WO 00079285, WO 00078455, WO 00069560, WO 98007019, WO 98053311); Ämic AB (WO 03024597, WO 04104585, WO 03101424 etc) etc. In less preferred variants the flow path may be in the form of tubes connecting various functional units such as the reaction cavity, mixing cavity, valve functions etc. In still other variants the flow path is defined by some kind of bibulous material/porous material through which liquid transport can take place by capillary force, for instance various kinds of conventional test strips.

The flow path is typically in the microformat, i.e. has dimensions and/or is capable of handling liquid volumes of the sizes discussed under "Preferred flow paths".

A suitable flow path typically comprises one or more reaction cavities one of which **(104a-h)** contains the above-mentioned solid phase that exhibits an immobilizing group or R1 in immobilized form. Other reaction cavities may be used for reaction between soluble reactants including reactants immobilized to suspended particles. There may also be mixing functions that may be in the form of cavities. A mixing cavity may coincide with a reaction cavity. These latter types of cavities/functional units typically are positioned upstream of a cavity containing the solid phase.

The reaction cavity **(104a-h)** is defined as the part of the flow path **(101a-h)** where the solid phase carrying R1 or an immobilizing group is present. The reaction cavity may be part of a larger reaction chamber.

The reaction cavity **(104a-h)** is typically in the microformat, i.e. has at least one cross-sectional dimension that is $\leq 1,000\ \mu m$, such as $\leq 500\ \mu m$ or $\leq 200\ \mu m$ (depth and/or width) and is then called microcavity. The smallest cross-sectional dimension is typically $\geq 5\ \mu m$ such as $\geq 25\ \mu m$ or $\geq 50\ \mu m$. The total volume of the reaction cavity **(104a-h)** in preferred flow paths is typically in the nl-range, such as $\leq 5,000$ nl, such as 1,000 nl or $\leq 500$ nl $\leq 100$ nl or $\leq 50$ nl or $\leq 25$ nl.

The term "flow conditions" means that the reaction between a solid phase and a soluble immobilizable reactant, such as immobilizable reactant R1, the Ig-analyte and an immobilizable complex that comprises R1 and the Ig-analyte while the liquid aliquot that comprises immobilizable reactant is continuously flowing through the reaction cavity/solid phase for the period of time during which the reaction takes place. The flow rate used may be adapted to provide non-diffusion limiting conditions or diffusion-limiting conditions for the reaction. Flow rates providing non-diffusion limiting conditions typically result in an enrichment (peak) of captured reactant in an upstream section of the solid phase.

The appropriate flow rate through the porous bed depends on a number of factors: The affinity between a solid phase bound reactant (e.g. R1) and the soluble reactant, (e.g. the Ig-analyte); b) kind of Ig-analyte; c) dimension of the reaction cavity (volume, length etc); d) kind of solid phase (material, porosity, bed or coated inner wall etc); and etc.

For flow paths that are in the microformat the flow rate typically should give a residence time of $\geq 0.010$ seconds such as $\geq 0.050$ sec or $\geq 0.1$ sec for the soluble reactant to pass the reaction cavity/solid phase, e.g. for a liquid aliquot containing the Ig-analyte passing a solid phase that exhibits R1. The upper limit for residence time is typically < 2 hours such as < I hour or < 15 min or < 5 min or < 1 min, or even shorter such as < 45 seconds. Illustrative flow rates are within 0.001-10,000 nl/sec, such as 0.01-1,000 nl/sec or 0.01-100 nl/sec or 0.1 - 10 nl/sec. These flow rate intervals may primarily be useful for solid phase volumes in the range of 1-1,000 nl, such as 1-200 nl or 1-50 nl or 1-25 nl. Residence time refers to the time it takes for a liquid aliquot to pass the solid phase. Optimization typically will require experimental testing on each particular system/format/reactant pair.

The liquid flow through the solid phase can be driven by in principle any kind of forces, e.g. electrokinetically or non-electrokinetically created forces. Centrifugal force possibly combined with capillary force for flow paths in microfluidic devices. See further under the heading "Preferred flow paths".

**Preferred flow paths (primarily microfluidic devices)**

A microfluidic device is a device that comprises one, two or more microchannel structures **(101a-h)** in which one or more liquid aliquots/samples that have volumes in the $\mu L$-range, typically in the nanolitre (nL) range transported and/or processed. At least one of these aliquots/samples contains one or more reactants selected amongst the Ig-analyte, a reagent such as detectable reactant R2, immobilizable reactant R1, an immobilizable complex formed in the assay, buffers and/or the like. The μL-range contemplates volumes ≤ 1000 μL, such as ≤ 100 μL or ≤ 10 μL and includes the nL-range that has an upper end of 5 000 nL but in most cases relates to volumes ≤ 1 000 nL, such as ≤ 500 nL or ≤ 100 nL. For an Ig-analyte that is present in the aliquot to be processed in high concentration, such as > 100 mg/L aliquots of even smaller volumes are concerned, e.g. ≤ 50 nl, such as ≤ 40 nL. The nL-range includes the picolitre (pL) range. A microchannel structure comprises one or more cavities and/or conduits that have a cross-sectional dimension that is ≤ $10^3$ μm, preferably ≤ $5 \times 10^2$ μm, such as ≤ $10^2$ μm.

A microchannel structure **(101a-h)** thus may comprise one, two, three or more functional units selected among: a) inlet arrangements **(102,103a-h)** comprising for instance an inlet port/inlet opening **(105a-b,107a-h),** possibly together with a volume-defining unit **(106a-h,108a-h)** (for metering liquid aliquots to be processed within the device), b) microconduits for liquid transport, c) reaction microcavities **(104a-h);** d) mixing microcavities/units; e) units for separating particulate matters from liquids (may be present in the inlet arrangement), f) units for separating dissolved or suspended components in the sample from each other, for instance by capillary electrophoresis, chromatography and the like; g) detection microcavities; h) waste conduits/microcavities **(112,115a-h);** i) valves **(109a-h,110a-h); j)** vents **(116a-i)** to ambient atmosphere; liquid splits (liquid routers) etc. A functional unit may have several functionalities, e.g. microcavity **(114a-h)** may be used both for performing reactions and for measurement/detection.

The reaction microcavity **(104a-h)** that is intended for the solid phase is typically part of a larger microchamber **(114a-h).** The reaction microcavity **(104a-h)** is typically positioned in close association with an outlet end of the microchamber **(114a-h).**

Various kinds of functional units in microfluidic devices have been described by Gyros AB/Amersham Pharmacia Biotech AB: WO 99055827, WO 99058245, WO 02074438, WO 02075312, WO 03018198, W0 04103890, WO 05032999, WO 05094976, WO 05072872, PCT/SE2005/001887; Tecan/Gamera Bioscience WO 01087487, WO 01087486, WO 00079285, WO 00078455, WO 00069560, WO 98007019, WO 98053311 etc. Included in this list are corresponding issued US patents and published US patent applications.

In advantageous forms a reaction microcavity **(104a-h)** intended for a solid phase is connected to one or more inlet arrangements (upstream direction) **(102,103a-h),** each of which comprises an inlet port **(105a-b,107a-h)** and at least one volume-defining unit **(106ah,108a-h).** One kind of inlet arrangement **(103a-h)** is connected to only one microchannel structure **(101a-h)** and/or reaction microcavity **(104a-h)** (individual inlet). Another kind of inlet arrangement **(102)** is common to all or a subset **(100)** of microchannel structures **(101a-h)** and/or microcavities **(104a-h).** The latter variant comprises a common inlet port **(105a-b)** that typically is combined with a distribution manifold that has one volume-defining unit/volume-metering microcavity **(106a-h)** for each microchannel structure/microcavity **(101a-h/104a-h)** of the subset **(100).** In both variants, each of the volume-defining units **(106a-h,108a-h)** including their volume-metering microcavities **(106a-h,113a-h)** in turn is communicating with downstream parts of its microchannel structure **(101a-h),** e.g. the microcavity **(104a-h).** Each volume-defining unit/volume-metering microcavity **(106a-h,108a-h/106a-h,113a-h)** typically has a valve **(109a-h,110a-h)** at its outlet end. This valve is typically passive, for instance utilizing a change in chemical surface characteristics at the outlet end, such as a boundary between a hydrophilic and hydrophobic surface (hydrophobic surface break) (WO 99058245, WO 2004103890, WO 2004103891 and or in geometric/physical surface characteristics (WO 98007019 (Gamera)).

The volumes that are to be defined/metered are volumes of liquid aliquots to be transported and processed further downstream in the microchannel structures. The volume of a metering microcavity (e.g. **106a-h,113a-h** with preference for those that are connected to an individual inlet port) used for an aliquot that contains the Ig-analyte in high concentration as discussed above has preferably a volume of ≤ 200 nl, such as ≤ 100 nl or ≤ 50 nl or ≤ 30 nl.

Typical inlet arrangements with inlet ports, volume-defining units, distribution manifolds, valves etc have been presented in WO 02074438, WO 02075312, WO 02075775 and WO 02075776 (all Gyros AB).

Each microchannel structure has at least one inlet opening **(105a-b,107a-h)** for liquids and at least one outlet opening for excess of air (vents) **(116a-i,112)** and possibly also for liquids (circles in the waste channel **(112)).**

The microfludic device used in the invention contains a plurality of microchannel structures, e.g. ≥ 10, e.g. ≥ 25 or ≥ 90 or ≥ 180 or ≥ 270 or ≥ 360 microchannel structures. The upper limit is typically ≤ 1000, such as ≤ 500.

Different principles may be utilized for transporting the liquid within the microfluidic device/microchannel structures between two or more of the functional units. Inertia force may be used, for instance by spinning the disc as discussed in the subsequent paragraph. Other useful forces are capillary forces, electrokinetic forces, non-electrokinetic forces such as capillary forces, hydrostatic pressure *etc.*

The microfluidic device typically is in the form of a disc. The preferred formats have an axis of symmetry (CD) that is perpendicular to or coincides with the disc plane, where n is an integer ≥ 2, 3, 4 or 5, preferably ∞ ($C_\infty$). In other words the disc may be rectangular, such as square-shaped and other polygonal forms but is preferably circular. Spinning the device around a spin axis that typically is perpendicular or parallel to the disc plane may create the necessary centrifugal force. Variants in which the spin axis is not perpendicular to a disc plane are given in WO 04050247 (Gyros AB).

The preferred devices are typically disc-shaped with sizes and/or forms similar to the conventional CD-format, e.g. sizes that are in the interval from 10% up to 300 % of a circular disc with the conventional CD-diameter (12 cm).

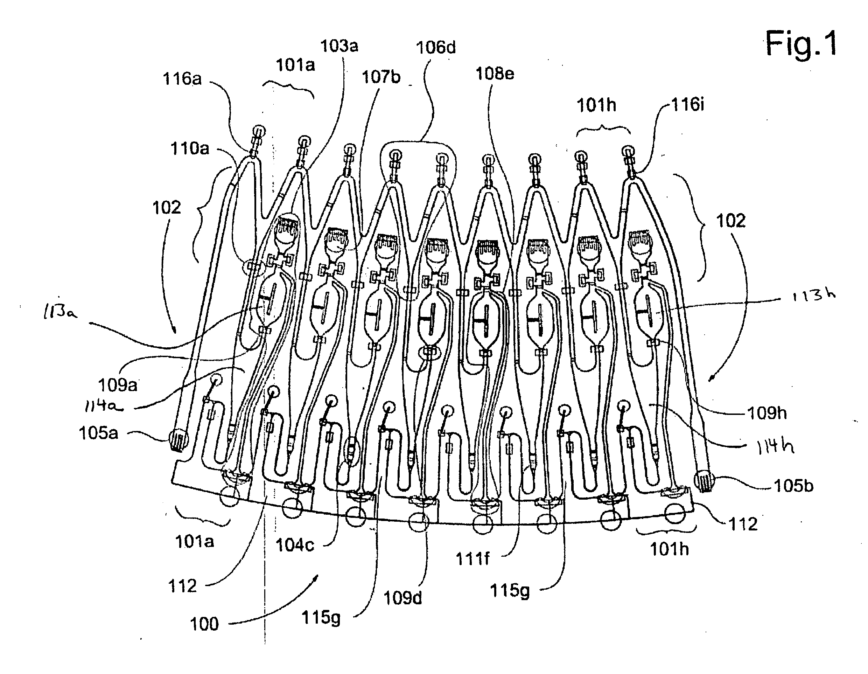

In the context of the invention the terms "wettable" (hydrophilic) and "non-wettable" (hydrophobic) of inner surfaces in a microchannel structure contemplate that a surface has a water contact angle $\leq 90°$ or $\geq 90°$, respectively. In order to facilitate efficient transport of a liquid between different functional parts of a microchannel structure, inner surfaces of the individual parts should primarily be wettable, preferably with a contact angle $\leq 60°$ such as $\leq 50°$ or $\leq 40°$ or $\leq 30°$ or $\leq 20°$. These wettability values apply for at least one, two, three or four of the inner walls of a microconduit. In the case one or more of the inner walls have a higher water contact angle, for instance is hydrophobic, this can be compensated for by a more wettable surfaces of one or more of the other inner wall(s). The wettability and the conduit dimensions, in particular in inlet arrangements, should be adapted such that an aqueous liquid to be used will be able to fill up an intended microcavity/microconduit by capillarity (self suction) once the liquid has started to enter the cavity/microconduit (the microcavity/microconduit is hydrophilic). A hydrophilic inner surface in a microchannel structure may comprise one or more local hydrophobic surface breaks in a hydrophilic inner wall, for instance for introducing a passive valve, an anti-wicking means, a vent solely function as a vent to ambient atmosphere etc (rectangles in **figure 1**). See for instance WO 99058245, WO 02074438, US 20040202579, WO 2004105890, WO 2004103891 (all Gyros AB).

Typical microchannel structures for formats that comprise mixing and/or incubation of soluble reactants (e.g. R1 in immobilizable form, R2 in detectable form, Ig-analyte, and other reactants) to produce an immobilizable affinity complex in an amount that is a function of the amount of analyte in a sample has been described in PCT/SE2005/001887 (Gyros Patent AB) and corresponding regular US application "Microfluidic assays and microfluidic devices" filed in December 2005. See also WO 02075312 (Gyros AB) In these kinds of microchannel structures there is typically a first mixing function upstream of the microcavity containing the solid phase and therebetween possibly a first incubation microcavity that may or may not at least partly coincide with the first mixing function. The mixing function may contain one, two or more inlets depending on the number of liquids and/or reactants that are to be mixed. The mixture obtained is transported downstream to the microcavity containing the solid phase possibly via the first incubation microcavity in which reactants in the mixture can react with each other to form an affinity complex before further transport into the microcavity containing the solid phase where the complex can be immobilized. Upstream of the first mixing function and in fluid communication with one or more of the inlets of this mixing function there may be one or more additional mixing functions each of which may or may not be associated with an incubation microcavity in the same manner as the first mixing function is associated with the first incubation microcavity. There may also be additional mixing functions, possibly combined with incubation microcavities connected to the flow path between the first mixing function and the microcavity containing the solid phase, for instance upstream and/or downstream of the first incubation microcavity. These additional mixing functions/incubation microcavities typically occur as branches of the flow path between the microcavity containing the solid phase and the first mixing function. Inlets of this kind of microchannel structure typically have volume-defining units at their inlets for on-device metering of liquid aliquots to be processed within the structure. A volume-defining unit may be associated with an individual inlet or with an inlet common to two or more structures, such as in a distribution manifold. Compare inlet arrangements **(103a-h)** and **(102),** respectively, in figure 1.

**Samples**

The liquid samples transported and processed in a microchannel structure are typically aqueous and may be diluents, wash liquids and/or liquids containing a reactant such as the Ig-analyte and/or a reagent, such the immobilized or immobilizable R1 and detectable R2.

An analyte sample introduced into a microchannel structure and/or into the microcavity containing the solid phase may be an unprocessed Ig-containing biological fluid sample or may derive from such a fluid sample. In preferred variants the sample containing the Ig-analyte is used in unprocessed undiluted form. The terms "unprocessed" and "undiluted" in this context includes that the sample have been cleared from particulate matters and other substances that may cause clogging or agglomeration and/or low dilutions such as dilution factors $\geq 1:5$ such as $\geq 1:3$. The term "biological fluid" contemplates any fluid that contains a bio-organic compound, in particular Ig. An Ig-containing biological fluid may be a cell culture supernatants homogenates and lysates, tissue homogenates, blood and various blood fractions such as serum or plasma, lymph, etc as well as various liquid preparations containing.

The volumes of the samples and concentration of an Ig-analyte in a sample to be used are discussed elsewhere in this specification. A growing and important subsegment of biotherapeutics is the field of therapeutic monoclonal antibodies. There are approximately 20 products that have acquired regulatory approval, 100-150 candidate antibodies in clinical development and another several hundreds in pre-clinical development. During 2005 the entire business comprised sales for approximately, 13 BUSD and is expected to increase to 30 BUSD in 2010.

The business has to adhere to compulsory regulatory requirements regarding efficacy and adverse effects that transform into product requirements of various types (dose, half-life, immunogenicity, specificity, affinity, carbohydrate composition etc). Another requirement is that the product should contain < 1 % of aggregates, i.e. dimers, trimers and larger aggregates, in order to reduce some of the potential adverse effects that may occur upon administration of drug (e.g. complement activation) and/or increased immunogenicity (aggregates are considered to be more immunogenic).

Aggregates of monoclonal antibodies may be formed due to a number of reasons:

The inherent properties of the antibody is prone to cause aggregation (e.g. antigen binding site is very hydrophobic)

Manufacturing conditions may induce aggregation e.g. due to aberrant carbohydrate composition

Purification procedures may induce denaturation of monoclonal IgG that may induce aggregation

Freeze-thawing cycles

If the therapeutic antibody shall be administered subcutaneously the volume that can be administered is limited to a few ml. Therefore, in order to administer the required dose of monoclonal antibody, the concentration has to be very high (typically in the range of 100-200 g/L). Under these circumstances the risk for formation of IgG aggregates is high.
There are several methods that can be used for analyse sample for contents of IgG aggregates (gel electrophoresis, ultracentrifugation, size exclusion chromatography, various optical methods such as turbidimetry, nephelometry or dynamic light scattering (DLS) (see references). One problem with size exclusion chromatography is that very large aggregates may not even enter the column used for separation and stay at the top of the column escaping detection of aggregates. Another is that small aggregates that are only weakly associated may become dissociated during the separation procedure.
As always each method has its pro's and con's. Some can analyse aggregates in native samples, other require purified samples in combination with orthogonal methods for IgG quantification, e.g. size exclusion chromatography. Ideally, analytical procedures should be applicable for samples irrespective of their status of purity, for instance in cell supernatants as well as after purification procedures that may induce aggregation. Another aspect is that the capacity of the method to analyse many samples during short periods of time, preferentially in parallel. Yet another aspect is the capability of the method to disclose also small proportions of aggregates in samples (assay sensitivity for aggregates).
In the era of monoclonal antibodies it has become easier to design assays for aggregates of proteins. Using the sandwich assay principle with the same capturing and detecting antibody, truly <u>monomeric</u> proteins in which epitopes used for immunoassay is only expressed once, will not be able to generate a response from an assay using two identical monoclonals. Only aggregated protein will be detected in the assay.
In contrast, immunoglobulins consist of 2 heavy and 2 light chains forming a tetramer in the native protein, represents a more complex structure and could in this perspective be described as "dimers of heterodimers". Thus any epitope on an immmunoglobulin is always expressed in "duplicate" on the molecular surface on the <u>monomeric</u> protein. This complicates the analysis of aggregates of immunoglobulins using monoclonal reagents in sandwich immunoassay. This proposal addresses a suggested procedure for analysis of IgG aggregates in therapeutic monoclonal antibody preparations using Gyrolab Bioaffy. The idea is described more in detail below.
We have designed assays for quantification of IgG based on capturing of IgG to biotinylated Fragment z (Fz), a commercially available derivative of protein A. The reactivity of Fz (mutated from region B of protein A) is solely directed against the region between $C_2H$ and $C_3H$ of the Fc portion of IgG from various species. In theory Fz can independently interact with the two heavy chains. Thus one should expect that an assay based on capturing Fz as well as detecting Fz shall generate signal for monomeric IgG.
Dissecting the molecular properties of biotinylated Fz (commercially available from Affibody) it is composed of 2 binding regions and the biotin molecule attached to the Fz through disulphide conjugation. A graphical representation can be designed as follows:

$$B \; \boxed{\; F_z \;|\; F_z \;} \qquad\qquad\qquad (1)$$

Fz is also commercially available in the following format:

$$( \; 2)$$

$$\boxed{F_z \;|\; F_z} \; S-S \; \boxed{F_z \;|\; F_z}$$

In the Fz:Fz assay we have used so far the "dimeric Fz" has been used for immobilisation and ALEXA labelled "tetrameric Fz" as detecting reagent. In a preferred version of the set-up it is likely that using the tetrameric version on the solid phase and the dimeric version as detecting reagent would probably enhance the selectivity for detection of aggregates of IgG Comparing the Fz:Fz assay format with an assay based on capturing Fz and detecting F(ab')$_2$ fragments of anti-human IgG gave different results, particularly in the low end of the concentration range of the two assays. Another observation made was that when analysing Fc fragments of human IgG, the Fz:Fz assay gave un-proportional strong response compared to the Fz:F(ab')$_2$ assay. Fc fragments are prone to form aggregates and can be readily crystallized (c in Fc stands for "crystallizable").

These assays have been used in combination with Bioaffy 20 HC. The HC particle may be of importance for the performance of the analysis. Thus, at least in the low end of the concentration range, large amounts of Fz immobilized Fz in the column will increase the probability that both heavy chains of a monomeric IgG molecule will react with excess amount of immobilized Fz locally available in the column. The consequence is that that it will be more difficult to incorporate the signal generating Fz-ALEXA into the monomeric protein, hence the monomeric protein will escape undetected under these circumstances, at least in the low concentration range of the measuring range. This principle may be even further emphasized by using "tetrameric Fz" on the solid phase and "dimeric Fz" as detecting reagent, an experiment not yet performed.

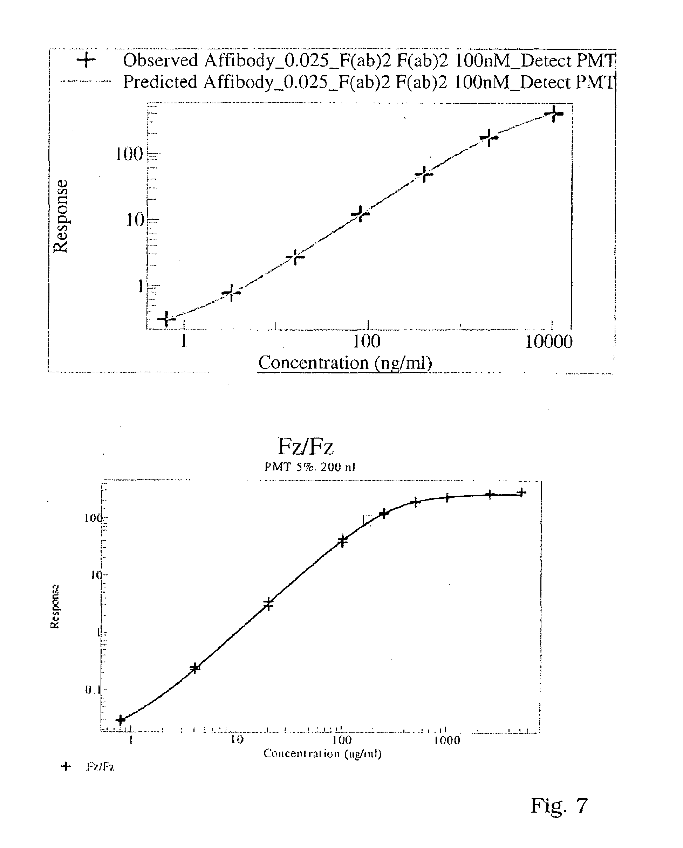

Until now the Fz:F(ab')$_2$ assay has been designed to quantitate IgG in as high concentration as possible. Roughly, the working range of the assay is 1-1200 ug IgG/ml (20 nl sample volume). In order to achieve this range the detecting antibody has to be mixed with non-labelled antibody that shifts the working range towards higher concentrations. When using only labelled antibody as detecting reagent the working range shifts towards low concentrations covering a range of roughly 2-10000 ng/ml (200 nl sample volume,). The corresponding assay range using the Fz:Fz assay is approximately 1-200 ug/ml (200 nl sample volume) as shown in Fig 7.

Using truly monomeric IgG as reference in the form of a reference curve and samples that contain various proportions of IgG aggregates that are analysed in:

An assay R1:R2 (e.g. Fz:F(ab)$_2$) quantifiying the concentration of IgG, that is essentially independent of the proportions of monomeric and polymeric IgG, e.g. the assay gives proportional response to the contents of IgG in the sample and
An assay R3:R4 (e.g. Fz:Fz) that gives a biased response in relation to the relative amount of polymeric IgG in the sample and
Calculating the ratio of concentrations determined by the two methods thereby incorporating the different behaviour of truly monomeric and aggregated IgG, respectively, in the two assays in the final result.

The proportion of polymeric IgG in a liquid sample is calculated with a truly monomeric IgG as a reference i.e., responses for assays Fz:F(ab')$_2$ and Fz:Fz are both tested against a truly monomeric sample and a sample to be identified.

The two assays should preferably be run in the same CD using high capacity particles in order to simplify the evaluation of the relative ratio of polymeric to monomeric protein in the sample.

In another embodiment according to the present invention any reagent broadly reacting with IgG, e.g., protein A or protein G, may be used instead of F(ab')$_2$ (R2) in the assay. In order to enhance the binding capacity for IgG, multiple binding molecules of Fz may be provided on the surface in the microchannel or on the beads in the microchannel.

In still another embodiment according to the present invention R1, R3 and R4 may be any one of the following affinity reactants, which react with the Fc part of IgG:

Fz which is a affibody
Fc binding function of protein G
Protein H
Synthetic binders with specificity to IgG (Fc)
Fab fragments
ScFv
V-domains
Bispecific scFv
Nanobodies derived from cameloid HC antibodies
Anticalins derived from lipocalins
Minobodies
Diabodies
Triabodies
Tertrabodies

In a further embodiment according to the present invention R1, R3 and R4 may be any one of the following affinity reactants, which react with the F(ab'2) part of IgG:

CHγI binding functionality of protein G
Protein L
Synthetic binders with specificity to IgG
Fab fragments
ScFv
V-domains
Bispecific scFv
Nanobodies
Anticalins derived from lipocalins
Minibodiers
Diabodies
Triabodies
Tetrabodies

## EXPERIMENTAL PART

### Microfluidic device and instrumentation

Two different microfluidic devices were used. One was Bioaffy CD microlaboratory commercially available (Gyros AB, Uppsala, Sweden) and detailed in figure 1 and in WO 04083108 (Gyros AB) and WO 04083109 (Gyros AB), except that the solid phase was tresyl-activated porous particles (10μm porous particles (TSKgel Tresyl-5PW, Tosoh Bioscience, Stuttgart, Germany) to which streptavidin had been preimmobilized in accordance with the manufacturer's instructions. Each of the structures had an individual metering microcavity **(113a-h)** of 200 nl. The other device was similar but each of the structures had an individual metering microcavities of 20 nl. The instrument used for processing was a Gyrolab Workstation equipped with laser fluorescence detector (Gyros AB, Uppsala, Sweden).

### REACTANTS AND LIQUIDS :

*Analyte (IgX):* Humant polyclonalt IgG. Stock solution 5 mg/ml

*Biotinylated fragment Z (fragment B (of protein A) mutated to abolish its $V_H$III binding ability:* Obtained from Affibody Technology, Stockholm, Sweden.

*Biotinylated recombinant protein G:* Commercially available with three IgG binding segments (peptide sequences) and devoid of the binding ability to other proteins that native protein G has). Biotinylation could be performed with EZ-Link-Sulfo-NHS-LC-Biotin/Sulfo-NHS-LC-biotin (Pierce, prod # 21335, Perbio Science UK Limited, Cheshire, United Kingdom). *Fluorophor-labelled fragment Z*: The fragment was obtained from Affibody Technology and labelled with Alexa fluorophor 647 monoclonal antibody labelling kit (A-20186, Molecular Probe) according the manufacturer's instruction except that twice the volume of liquid containing the reactive dye (Alexa fluorophor) was used. Incubation was allowed to proceed for three ours. Due to the very small size of fragment Z, the separation of labelled polypeptide was done with a Dialysis membrane (Pierce#69558). *Fluorophor-labelled Protein* G: The naked protein G was recombinantly produced and obtained from ???. Labelling was carried out by the use of Alexa fluorophor 647 monoclonal antibody labelling kit (A-20186, Molecular Probe) according the manufacturer's instruction.
*Wash liquid*: The wash associated with the capture step and the detection step was carried out with 1 x PBS 0.01 % Tween®.

*Dilution:*

The capture reagent (R1) is diluted in 1 x PBS containing 0.01 % BSA
The detection reagent is diluted in 1 x PBS containing 0.01% Tween®
The analyte (IgX, Ig-analyte) is diluted in PBS, 0.01% Tween®.

### ASSAY PROCEDURE

**Activation step 1:** The capturer (R1) was immobilized on the columns **(104a-h)** in the microchambers **(114a-**

**h)** by introduction of the biotinylated reagent (R1) into a common inlet port **(105a** or **b)** followed by spinning the device such that liquid in the metering microcavities **(106a-h)** was forced to pass into the microchambers **(114a-h)** and through the solid phase **(104a-h)** thereby introducing the capturer (R1) on each column.

**Step 1:** *Capture of Ig-analyte.* A diluted sample of the Ig stock solution was introduced into each of the individual inlet ports **(107a-h)** thereby filling up the metering microcavities **(113a-h,** respectively). The device was subsequently spinned thereby forcing the liquid in each filled-up metering microcavity to pass into its downstream microcavity **(114a-h)** and through the corresponding column **(104a-h)** where the analyte was captured.

**Step 2:** *Detection reactant R2.* Fluorophor labeled R2 was introduced via the common inlet port **(105a** or **b).** As in activation step and step 1 the device was subsequently spinned thereby forcing the liquid in each filled up metering microcavity **(106a-h)** to pass into its downstream microcavity **(114a-h)** and through the corresponding column **(104a-h)** where R1 was captured. The fluorescence from the columns was measured with different PMT settings before and after the introduction of R2 on the solid phases.

Separate wash steps were included before, between, and after each addition of R1, R2 and the analyte. Wash liquids were introduced via a common inlet port **(105a** or b). Compare the spin protocol given in the next paragraph.

**SPIN PROTOCOLS**

**Initial needle wash common,** Particle wash I, Particle wash spin 1, Particle wash 2 structure, Particle wash 2 common, Particle wash spin 2

**Capture reagent addition common inlet,** Capture reagent spin, Capture reagent wash 1,
Capture reagent wash spin 1, Capture reagent wash 2, Capture reagent wash spin 2 **Analyte addition individual inlets,** Analyte spin, Analyte wash 1, Analyte wash spin 1,
Analyte wash 2, Analyte wash spin 2

**CD alignment 1,** Detect background PMT 1 Detect background PMT 2 and Detect background PMT 3, Spin out

**Detection reagent additioncommon inlet:** Detection reagent spin, Detection reagent wash 1,
Detection reagent wash spin 1, Detection reagent wash 2, Detection reagent wash spin 2, Detection reagent wash 3, Detection reagent wash spin 3, Detection reagent wash 4,
Detection reagent wash spin 4

**CD alignment 2,** Detect PMT 1, Detect PMT 2, Detect PMT 3

Wash liquids via common inlet.

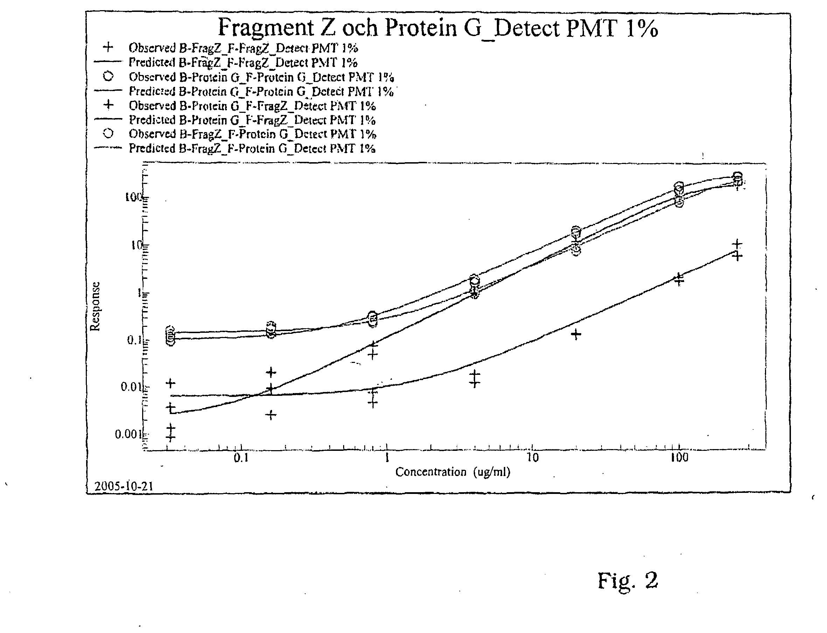

The results of the experiments are illustrated in figures 2-6.

**Figure 2.** Different combinations of fragment Z and recombinant protein G. (1) biotinylated fragment Z (R1) and Fluorophor-labelled fragment Z (R2), (2) biotinylated protein G (R1) and fluorophor-labelled protein G (R2), (3) biotinylated protein G (R1) and fluorophor-labelled fragment z (R2), and (4) biotinylated fragment Z (R1) and Fluorophor-labelled protein G (R2). Analyte samples 200 nl. The experiments illustrate that different combinations of IgG binding constructs will give different performance of the assay with respect to parameters such as dynamic range and detection limit.

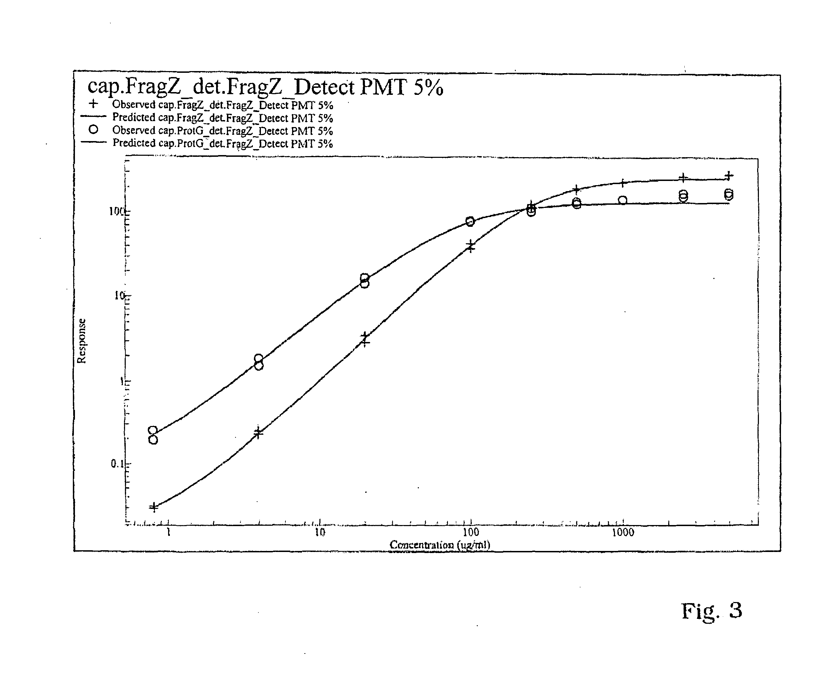

**Figure 3.** Comparison between (1) biotinylated fragment Z (R1) and Fluorophor-labelled fragment Z (R2), (2) biotinylated protein G (R1) and fluorophor-labelled protein G (R2). Analyte samples 200 nl.

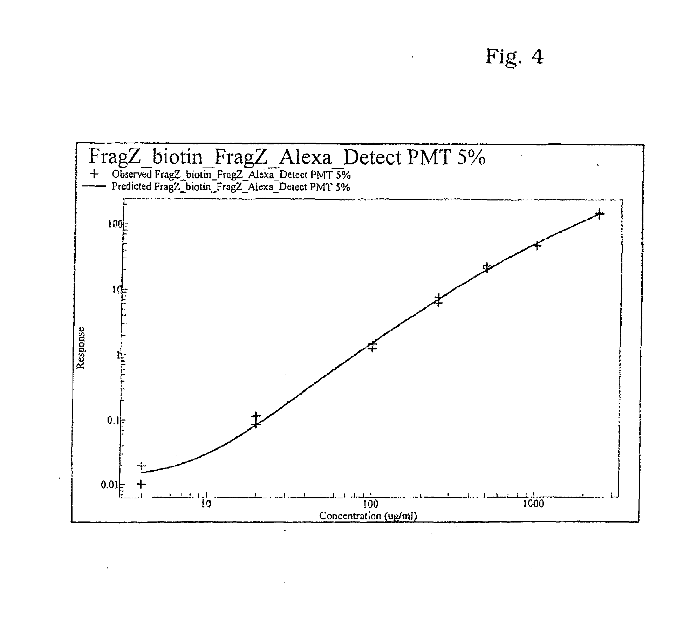

**Figure 4.** Biotinylated fragment Z (R1) and Fluorophor-labelled fragment Z (R2). Analyte samples 200 nl. IgG-Fluorescence minimized by the use of an IgG-filter. Note the dynamic range (at least three orders of magnitude).

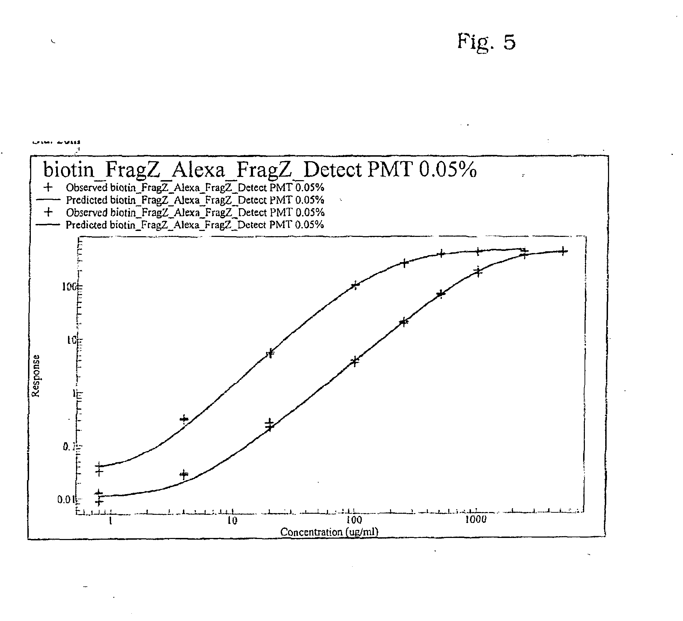

**Figure 5.** Comparison between different volumes of analyte samples. 200 nl (1) and 20 nl (2). Biotinylated fragment Z (R1) and Fluorophor-labelled fragment Z (R2). Without IgG-filter.

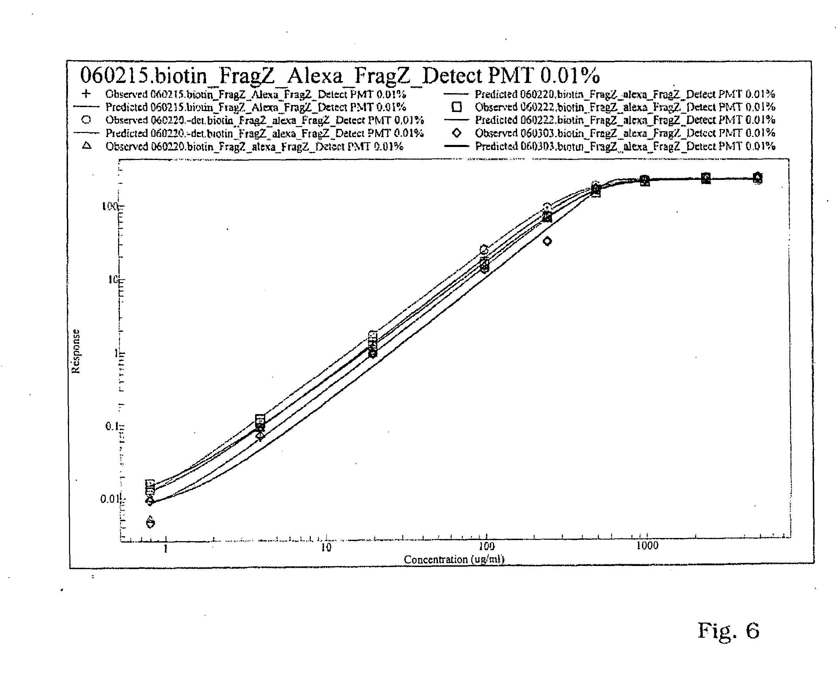

**Figure 6.** Exclusion of wash steps when using biotinylated fragment Z as R1 and fluorophor-labelled fragment Z as R2. Analyte samples 20 nl. The different graphs corresponds to experiments in which a wash step was excluded: (1) the spin protocol given above, (2) exclusion of one detector wash, (3) exclusion of one capture wash, (4) exclusion of one capture wash and one detector wash, (5) exclusion of one capture wash and two detector wash. The graphs inticate that wash steps can be excluded thereby reducing the time for running one device. In total the reduction for one device containing 104 structures was from 53 minutes and 29 seconds to 39 minutes and 15 seconds. If variant 1 run on a device having individual metering microcavities of 200 nl is compared with variant 5 (20 nl), the reduction will be from 60 min 7 seconds to 39 minutes and 15 seconds.

Fig 7. Below two illustrations of how the two different assays respond in terms of measuring range for seemingly monomeric IgG in Bioaffy 200. Please note that the measuring range differ by a factor of approximately 100-1000 times in the two assays.

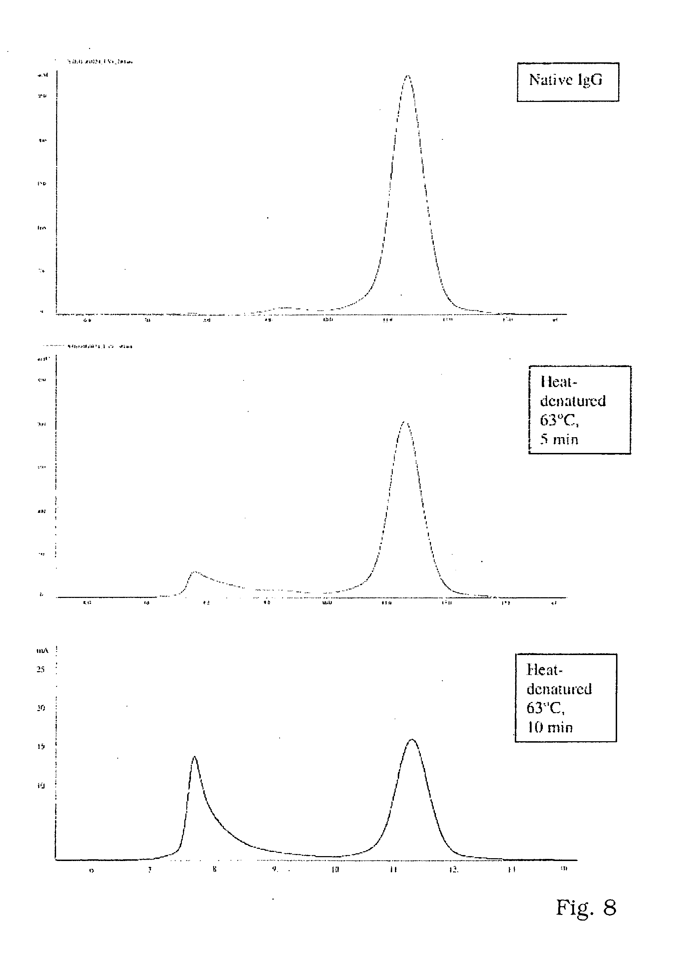

Fig 8. Gelfiltration of native IgG (IgG1$\kappa$ at 2 mg/ml in PBS, pH7.4) (top panel), IgG that was heat aggregated at 63°C for 5 min (middle panel), and IgG that was heat aggregated for 10 min at 63°C (bottom panel), respectively, on a 1.5 x 30 cm Superdex 200 column. Fractions collected were analysed in the Fz:Fz and Fz:F(ab')$_2$ assays for

concentration of IgG

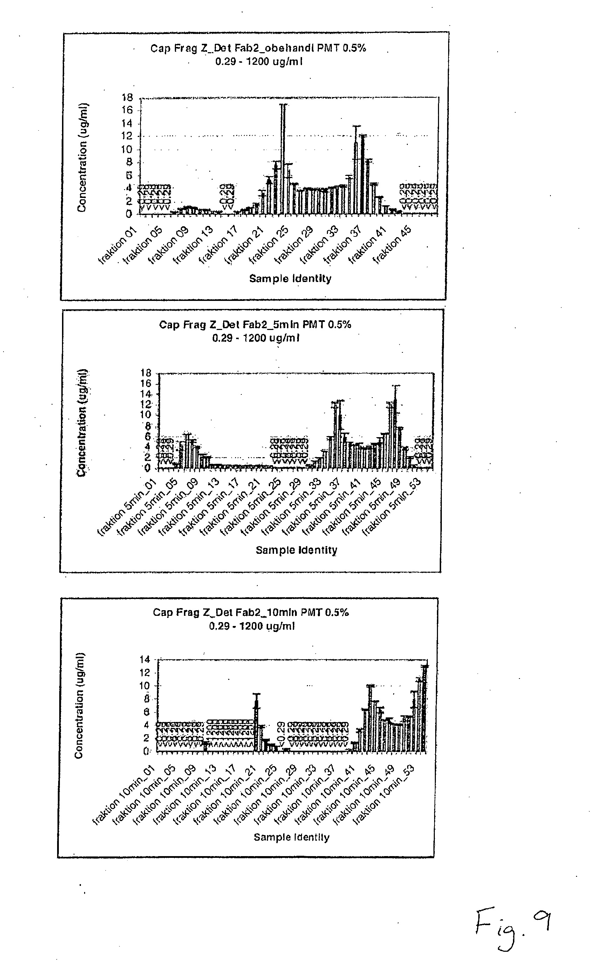

Fig 9. Fractions from gelfiltration on Superdex 200 of 3 different samples (native IgG, IgG that were pretreated at 63°C for 5 min and 10 min, respectively) were analysed in Fz:F(ab')$_2$ and Fz:Fz assay, respectively. The concentration of IgG in fractions 25-35 is grossly underestimated in this assay.

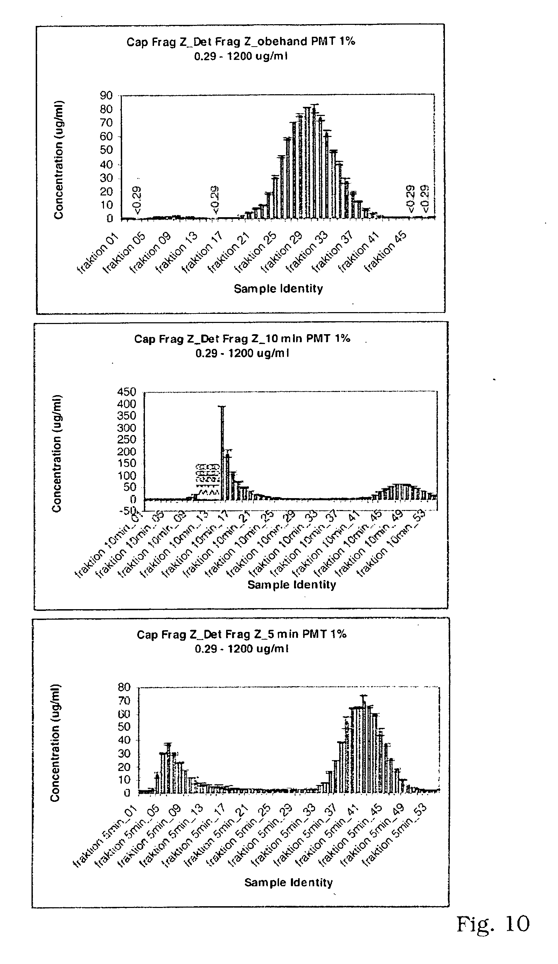

Fig 10. Fractions from gelfiltration on Superdex 200 of 3 different samples (native IgG, IgG that were pretreated at 63°C for 5 min and 10 min, respectively) were analysed in Fz:F(ab')$_2$ and Fz:Fz assay, respectively.

Certain innovative aspects of the invention are defined in more detail in the appending claims. Although the present invention and its advantages have been described in detail, it should be understood that various changes, substitutions and alterations can be made herein without departing from the spirit and scope of the invention as defined by the appended claims. Moreover, the scope of the present application is not intended to be limited to the particular embodiments of the process, machine, manufacture, composition of matter, means, methods and steps described in the specification. As one of ordinary skill in the art will readily appreciate from the disclosure of the present invention, processes, machines, manufacture, compositions of matter; means, methods, or steps, presently existing or later to be developed that perform substantially the same function or achieve substantially the same result as the corresponding embodiments described herein may be utilized according to the present invention. Accordingly, the appended claims are intended to include within their scope such processes, machines, manufacture, compositions of matter, means, methods, or steps.

## Claims

1. A method for quantitative determination of a proportion of polymeric immunoglobulin (Ig) of class IgG in a liquid sample comprising the steps of:

   a) forming a first complex in which IgG is sandwiched between two affinity reactants 1 and 2 (R1 and R2, respectively), where said complex gives proportional response to a concentration of IgG in the sample independently of being in monomeric or polymeric form,

   b) forming a second complex in which IgG is sandwiched between two affinity reactants 3 and 4 (R3 and R4, respectively), where said complex gives biased response to a concentration of polymeric IgG relative to monomeric IgG,

   c) calculating the ratio of said concentrations of said first complex and said second complex by using truly monomeric IgG as a reference in order to determine the proportion of polymeric IgG in said liquid sample.

2. The method according to claim 1, wherein affinity reactant R1, R3 and R4 have affinity to the FC domain of IgG, and affinity reactant R2 is a binder with a different specificity for IgG compared to R1, R2 and R3.

3. The method according to claim 2, wherein R2 is a reagent broadly reacting with IgG.

4. The method according to claim 3, wherein R2 is F(ab')$_2$.

5. The method according to claim 2, wherein at least one of R1, R3 and R4 is Fz.

6. The method according to any one of claim 1-5, **characterized in that** R1 and R3 are immobilized or immobilizable to a solid phase and R2 and R4 is analytically measurable.

7. The method according to claim 1-5, **characterized in that** at least one of R1 and R3 is provided immobilized in multiple form to a solid phase in the form of a porous bed in a reaction cavity of a flow path.

8. The method according to claim 1-5, **characterized in that** at least one of R1 and R3 is provided immobilized to a solid phase in the form of a porous bed in a reaction cavity of a flow path.

9. The method according to claim 1-5, **characterized in that** at least one of R1 and R3 is provided immobilized in a multiple form to a solid phase in the form of a porous bed in a reaction cavity of a flow path.

10. The method according to any one of claims 1-9, **characterized in that** the flow path is a microchannel structure of microfluidic device.

11. The method according to any one of claim 1-10, **characterized in that** said microchannel structure comprises

a) a first reaction microcavity containing a solid phase to which R1 is immobilized or immobilizable,
b) a first inlet arrangement for the introduction of said sample containing IgG and for the subsequent introduction of a liquid sample containing R2 in analytically measurable form,
c) a second reaction microcavity containing a solid phase to which R3 is immobilized or immobilizable, and
d) a second inlet arrangement for the introduction of said sample containing IgG and for the subsequent introduction of a liquid sample containing R4 in analytically measurable form.

12. The method claims 10, **characterized in that** a volume-metering unit is present in at least one of said microchannels and has a liquid metering microcavity that has a volume in the interval of 1-5000 nL for at least said first part, such as $\leq 1000$ nl or $\leq 500$ nl or $\leq 100$ nl or $\leq 50$ nl or $\leq 30$ nl.

13. The method of any of claims 1-12, **characterized in that** said sample containing IgG is undiluted and preferably contains a concentration of IgG that is in the interval of 1 $\mu$g/L - 10000 mg/L, such as 0.5 mg/L - 1000 mg/L.

14. The method of any of claims 1-13, **characterized in that** the sample containing IgG derives from a supernatant, a cell lysate, cell homogenate from a cell culture that have produced said IgG or a liquid containing IgG that has been produced by cell culturing.

15. The method according to any one of claims 1-14, **characterized in that** at least one of R2 and R4 is a monomer.

**Patentansprüche**

1. Verfahren zur quantitativen Bestimmung des Anteils an polymerem Immunglobulin (Ig) der Klasse IgG in einer flüssigen Probe umfassend die Schritte:

a) Bilden eines ersten Komplexes, in dem IgG zwischen zwei Affinitätsreaktanten 1 und 2 (R1 beziehungsweise R2) angeordnet ist, wobei der Komplex eine proportionale Antwort auf eine Konzentration an IgG in der Probe gibt, unabhängig davon, ob es in monomerer oder polymerer Form vorliegt,
b) Bilden eines zweiten Komplexes, in dem IgG zwischen zwei Affinitätsreaktanten 3 und 4 (R3 beziehungsweise R4) angeordnet ist, wobei der Komplex eine verzerrte Antwort auf eine Konzentration an polymerem IgG relativ zu monomerem IgG gibt,
c) Berechnen des Verhältnisses der Konzentrationen des ersten Komplexes und des zweiten Komplexes unter Verwenden von reinem monomeren IgG als Referenz, um den Anteil an polymerem IgG in der flüssigen Probe zu bestimmen.

2. Verfahren gemäß Anspruch 1, wobei die Affinitätsreaktanten R1, R3 und R4 Affinität zu der Fc-Domäne von IgG haben und Affinitätsreaktant R2 ein Bindemittel mit unterschiedlicher Spezifität für IgG im Vergleich zu R1, R2 und R3 ist.

3. Verfahren gemäß Anspruch 2, wobei R2 ein Reagens ist, welches in einem breiten Bereich mit IgG reagiert.

4. Verfahren gemäß Anspruch 3, wobei R2 F(ab')$_2$ ist.

5. Verfahren gemäß Anspruch 2, wobei mindestens einer von R1, R3 und R4 Fz ist.

6. Verfahren gemäß einem der Ansprüche 1 bis 5, **dadurch gekennzeichnet, dass** R1 und R3 immobilisiert oder immobilisierbar an eine feste Phase sind und R2 und R4 analytisch messbar sind.

7. Verfahren gemäß Ansprüchen 1 bis 5, **dadurch gekennzeichnet, dass** mindestens einer von R1 und R3 in multipler Form immobilisiert an eine feste Phase in Form eines porösen Bettes in einer Reaktionsvertiefung eines Fließpfads bereitgestellt wird.

8. Verfahren gemäß Ansprüchen 1 bis 5, **dadurch gekennzeichnet, dass** mindestens einer von R1 und R3 immobilisiert an eine feste Phase in Form eines porösen Bettes in einer Reaktionsvertiefung eines Fließpfads bereitgestellt wird.

**9.** Verfahren gemäß Ansprüchen 1 bis 5, **dadurch gekennzeichnet, dass** mindestens einer von R1 und R3 in multipler Form immobilisiert an eine feste Phase in Form eines porösen Bettes in einer Reaktionsvertiefung eines Fließpfads bereitgestellt wird.

**10.** Verfahren gemäß einem der Ansprüche 1 bis 9, **dadurch gekennzeichnet, dass** der Fließpfad eine Mikrokanalstruktur einer mikrofluiden Vorrichtung ist.

**11.** Verfahren gemäß einem der Ansprüche 1 bis 10, **dadurch gekennzeichnet, dass** die Mikrokanalstruktur umfasst:

a) eine erste Reaktionsmikrovertiefung enthaltend eine feste Phase, an die R1 immobilisiert oder immobilisierbar ist,
b) eine erste Einlassöffnungsanordnung zum Einbringen der Probe, die IgG enthält, und zum anschließenden Einbringen einer flüssigen Probe, die R2 in analytisch messbarer Form enthält,
c) eine zweite Reaktionsmikrovertiefung enthaltend eine feste Phase, an die R3 immobilisiert oder immobilisierbar ist, und
d) eine zweite Einlassöffnungsanordnung zum Einbringen der Probe, die IgG enthält, und zum anschließenden Einbringen einer flüssigen Probe, die R4 in analytisch messbarer Form enthält.

**12.** Verfahren gemäß Anspruch 10, **dadurch gekennzeichnet, dass** eine Volumenmesseinheit in mindestens einem der Mikrokanäle vorhanden ist und eine Mikrovertiefung zur Flüssigkeitsmessung aufweist, die ein Volumen im Bereich von 1-5000 nL für mindestens den ersten Anteil hat, wie ≤ 1000 nl oder ≤ 500 nl oder ≤ 100 nl oder ≤ 50 nl oder ≤ 30 nl.

**13.** Verfahren gemäß einem der Ansprüche 1 bis 12, **dadurch gekennzeichnet, dass** die Probe, die IgG enthält, unverdünnt ist und vorzugsweise eine Konzentration an IgG enthält, die im Bereich von 1 μg/L - 10000 mg/L ist, wie 0,5 mg/L - 1000 mg/L.

**14.** Verfahren gemäß einem der Ansprüche 1 bis 13, **dadurch gekennzeichnet, dass** die Probe, die IgG enthält, von einem Überstand, einem Zelllysat, einem Zellhomogenisat von einer Zellkultur, die das IgG produziert haben, oder einer Flüssigkeit, die IgG enthält, welches durch Zellkultivierung produziert wurde, stammt.

**15.** Verfahren gemäß einem der Ansprüche 1 bis 14, **dadurch gekennzeichnet, dass** mindestens einer von R2 und R4 ein Monomer ist.

**Revendications**

**1.** Procédé pour la détermination quantitative d'une proportion d'immunoglobuline (Ig) polymère de la classe des IgG dans un échantillon liquide, comprenant les étapes consistant :

a) à former un premier complexe dans lequel l'IgG est en sandwich entre deux réactifs d'affinité 1 et 2 (R1 et R2, respectivement), ledit complexe donnant une réponse proportionnelle à une concentration d'IgG dans l'échantillon indépendamment du fait qu'elle est sous forme monomère ou polymère,
b) à former un second complexe dans lequel l'IgG est en sandwich entre deux réactifs d'affinité 3 et 4 (R3 et R4, respectivement), ledit complexe donnant une réponse biaisée à une concentration d'IgG polymère par rapport à une IgG monomère,
c) à calculer le rapport desdites concentrations dudit premier complexe et dudit second complexe en utilisant une IgG véritablement monomère en tant que référence afin de déterminer la proportion d'IgG polymère dans ledit échantillon liquide.

**2.** Procédé selon la revendication 1, dans lequel les réactifs d'affinité R1, R3 et R4 ont de l'affinité pour le domaine FC d'une IgG, et le réactif d'affinité R2 est un liant ayant une spécificité différente pour une IgG par rapport à R1, R2 et R3.

**3.** Procédé selon la revendication 2, dans lequel R2 est un réactif réagissant largement avec les IgG.

**4.** Procédé selon la revendication 3, dans lequel R2 est F(ab')2

**5.** Procédé selon la revendication 2, dans lequel au moins l'un parmi R1, R3 et R4 est Fz.

**6.** Procédé selon l'une quelconque des revendications 1 à 5, **caractérisé en ce que** R1 et R3 sont immobilisés ou immobilisables sur une phase solide et R2 et R4 sont dosables analytiquement.

**7.** Procédé selon les revendications 1 à 5,
**caractérisé en ce qu'**au moins l'un parmi R1 et R3 est fourni immobilisé sous forme multiple sur une phase solide sous la forme d'un lit poreux dans une cavité de réaction d'un chemin d'écoulement.

**8.** Procédé selon les revendications 1 à 5,
**caractérisé en ce qu'**au moins l'un parmi R1 et R3 est fourni immobilisé sur une phase solide sous la forme d'un lit poreux dans une cavité de réaction d'un chemin d'écoulement.

**9.** Procédé selon les revendications 1 à 5,
**caractérisé en qu'**au moins l'un parmi R1 et R3 est fourni immobilisé sous une forme multiple sur une phase solide sous la forme d'un lit poreux dans une cavité de réaction d'un chemin d'écoulement.

**10.** Procédé selon l'une quelconque des revendications 1 à 9, **caractérisé en ce que** le chemin d'écoulement est une structure à microcanaux d'un dispositif microfluidique.

**11.** Procédé selon l'une quelconque des revendications 1 à 10, **caractérisé en ce que** ladite structure à microcanaux comprend

a) une première microcavité de réaction contenant une phase solide sur laquelle R1 est immobilisé ou immobilisable,
b) un premier agencement d'entrée pour introduire ledit échantillon contenant une IgG et pour introduire ensuite un échantillon liquide contenant R2 sous forme dosable analytiquement,
c) une seconde microcavité de réaction contenant une phase solide sur laquelle R3 est immobilisé ou immobilisable, et
d) un second agencement d'entrée pour introduire ledit échantillon contenant une IgG et pour introduire ensuite un échantillon liquide contenant R4 sous forme dosable analytiquement.

**12.** Procédé selon la revendication 10, **caractérisé en ce qu'**un dispositif de dosage volumique est présent dans au moins l'un desdits microcanaux et a une microcavité de dosage de liquide qui a un volume dans l'intervalle de 1 à 5 000 nl pour au moins ladite première partie, comme $\leq$ 1 000 nl ou $\leq$ 500 nl ou $\leq$ 100 nl ou $\leq$ 50 nl ou $\leq$ 30 nl.

**13.** Procédé selon l'une quelconque des revendications 1 à 12, **caractérisé en ce que** ledit échantillon contenant une IgG est non dilué et contient de préférence une concentration d'IgG qui est dans l'intervalle de 1 $\mu$g/l à 10 000 mg/l, comme 0,5 mg/l à 1 000 mg/1.

**14.** Procédé selon l'une quelconque des revendications 1 à 13, **caractérisé en ce que** l'échantillon contenant une IgG est issu d'un surnageant, d'un lysat cellulaire, d'un homogénat cellulaire d'une culture cellulaire qui ont produit ladite IgG ou d'un liquide contenant une IgG qui a été produite par culture cellulaire.

**15.** Procédé selon l'une quelconque des revendications 1 à 14, **caractérisé en ce qu'**au moins l'un parmi R2 et R4 est un monomère.

Fig.1

Fig. 2

cap.FragZ_det.FragZ_Detect PMT 5%

+   Observed cap.FragZ_det.FragZ_Detect PMT 5%
—— Predicted cap.FragZ_det.FragZ_Detect PMT 5%
O   Observed cap.ProtG_det.FragZ_Detect PMT 5%
—— Predicted cap.ProtG_det.FragZ_Detect PMT 5%

Fig. 3

Fig. 4

FragZ_biotin_FragZ_Alexa_Detect PMT 5%

+ Observed FragZ_biotin_FragZ_Alexa_Detect PMT 5%
—— Predicted FragZ_biotin_FragZ_Alexa_Detect PMT 5%

Fig. 5

biotin_FragZ_Alexa_FragZ_Detect PMT 0.05%

+ Observed biotin_FragZ_Alexa_FragZ_Detect PMT 0.05%
— Predicted biotin_FragZ_Alexa_FragZ_Detect PMT 0.05%
+ Observed biotin_FragZ_Alexa_FragZ_Detect PMT 0.05%
— Predicted biotin_FragZ_Alexa_FragZ_Detect PMT 0.05%

## 060215.biotin_FragZ_Alexa_FragZ_Detect PMT 0.01%

+ Observed 060215.biotin_FragZ_Alexa_FragZ_Detect PMT 0.01%
— Predicted 060215.biotin_FragZ_Alexa_FragZ_Detect PMT 0.01%
○ Observed 060220.-det.biotin_FragZ_alexa_FragZ_Detect PMT 0.01%
— Predicted 060220.-det.biotin_FragZ_alexa_FragZ_Detect PMT 0.01%
△ Observed 060220.biotin_FragZ_alexa_FragZ_Detect PMT 0.01%
— Predicted 060220.biotin_FragZ_alexa_FragZ_Detect PMT 0.01%
□ Observed 060222.biotin_FragZ_alexa_FragZ_Detect PMT 0.01%
— Predicted 060222.biotin_FragZ_alexa_FragZ_Detect PMT 0.01%
◇ Observed 060303.biotin_FragZ_alexa_FragZ_Detect PMT 0.01%
— Predicted 060303.biotin_FragZ_alexa_FragZ_Detect PMT 0.01%

Fig. 6

Fig. 7

Native IgG

Heat-
denatured
63°C,
5 min

Heat-
denatured
63°C,
10 min

Fig. 8

25

Fig. 9

Fig. 10

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 02075312 A
- WO 2004083109 A
- SE 06000071 W
- SE 06000072 W
- WO 99055827 A
- WO 99058245 A
- WO 02074438 A
- WO 03018198 A
- US 20030044322 A
- WO 01087487 A
- WO 01087486 A
- WO 00079285 A
- WO 00078455 A
- WO 00069560 A
- WO 98007019 A
- WO 98053311 A
- WO 03024597 A
- WO 04104585 A
- WO 03101424 A
- WO 04103890 A
- WO 05032999 A
- WO 05094976 A
- WO 05072872 A
- SE 2005001887 W
- WO 2004103890 A
- WO 2004103891 A
- WO 02075775 A
- WO 02075776 A
- WO 04050247 A
- US 20040202579 A
- WO 2004105890 A
- WO 04083108 A
- WO 04083109 A

### Non-patent literature cited in the description

- **Nilsson B et al.** *Protein Eng.,* 1987, vol. 1, 107-113
- **Nilsson J et al.** *Eur J Biochem,* 1994, vol. 224, 103-108
- **Jansson B et al.** *FEMS Immunology and Medical Microbiology,* 1998, vol. 20, 69-78
- **Derrick JP et al.** *J Mol Biol,* 1994, vol. 243, 906-918
- **Gülich S et al.** *Protein Eng,* 2002, vol. 15, 835-842
- **Kelley RF et al.** *Biochemistry,* 1992, vol. 31, 5434-5441
- **Sloan DJ et al.** *Protein Sci,* 1999, vol. 8, 1643-1648